# EUROPÄISCHE PATENTSCHRIFT

(11) **EP 0 511 234 B1**
(45) Veröffentlichungstag und Bekanntmachung des Hinweises auf die Patenterteilung: **12.04.1995**
(21) Anmeldenummer: 91901746.7
(22) Anmeldetag: 19.12.1990
(51) Int. Cl.: A61K 35/16, A61K 47/18, A61K 47/26, A61K 9/08

(54) **STABILE INJIZIERBARE LÖSUNGEN VON FAKTOR VIII**
STABLE INJECTIBLE SOLUTIONS FOR FACTOR VIII
SOLUTIONS STABLES INJECTABLES DU FACTEUR VIII

(30) Priorität: 19.01.1990 DE 4001451
(43) Veröffentlichungstag der Anmeldung: 04.11.1992
(73) Patentinhaber: Octapharma AG, 8866 Ziegelbrücke (CH)
(72) Erfinder: SCHWINN, Horst, D-3550 Marburg (DE)
(74) Vertreter: Werner, Hans-Karsten, Dr.Dipl.-Chem.
(86) Internationale Anmeldenummer: EP9002238
(87) Internationale Veröffentlichungsnummer: WO9110439

(56) Entgegenhaltungen:
- EP-A- 0 012 156
- EP-A- 0 018 561
- EP-A- 0 035 204
- EP-A- 0 077 870
- EP-A- 0 117 064
- EP-A- 0 137 428
- EP-A- 0 314 095
- WO-A-82/03871

## Beschreibung

Gegenstand der vorliegenden Anmeldung ist eine stabile injizierbare Lösung von Faktor VIII, ein Verfahren zu ihrer Herstellung und eine Verwendung.

Faktor VIII ist ein an der Blutgerinnung und an der Fibrinolyse beteiligter Gerinnungsfaktor des menschlichen Blutgerinnungssystems. Faktor VIII ist an der Bildung des Plasma-Prothrombin-Aktivators im endogenen Gerinnungssystem beteiligt.

Ein Mangel des Antihämophiliefaktors VIII führt zu einer krankhaften Störung des Blutgerinnungssystems in Form der Hämophilie. Die Restaktivität des Gerinnungsfaktors kann dabei auf wenige Prozent herabsinken. Zur Therapie der Hämophilie werden im Falle eines Faktor VIII-Defizits gerinnungsaktive Plasmakonzentrate wie Kryopräzipitat verwendet.

Bei der Verwendung dieses Gerinnungsfaktors als Therapeutikum ist jedoch zu beachten, daß dieses instabil ist, und Fibrinogen sowie unerwünschte Viren enhalten kann.

Es ist daher notwendig, den angereicherten Gerinnungsfaktor einem Reinigungs- und Stabilisierungsprozeß zu unterziehen.

Ein Verfahren zur Herstellung eines hochreinen, nicht infektiösen Antihämophiliefaktors wird in der Europäischen Patentanmeldung 88 118 478.2 vorgeschlagen. Dabei wird die an Faktor VIII angereicherte Fraktion durch eine Gelpermeationschromatographie mit Ionenaustauschermaterialien gewonnen. Die Aufarbeitung dieser Fraktion erfolgt danach ebenfalls durch ein gelpermeationschromatographisches Verfahren auf Basis von Ionenaustauschern.

Die Europäische Patentanmeldung 0 018 561 beschreibt ein Verfahren zur Stabilisierung von Blutgerinnungsfaktoren, insbesondere der Faktoren II, VIII, XIII und III sowie Plasminogen, durch Erhitzen auf 60 bis 70 °C in Gegenwart von Glycin und einem Saccharid bei einem pH-Wert zwischen 6,5 und 8, wobei das Erhitzen in Gegenwart von Saccharose in einer Konzentration von 20 bis 60 Gewichtsprozent und von Glycin in einer Konzentration von 1,0 bis 3,0 mol/l vorgenommen wird.

Das so gewonnene an Gerinnungsfaktoren angereicherte Konzentrat wird nach der Reinigung und Stabilisierung steril filtriert und/oder lyophilisiert. Lyophilisierte Präparate von Faktor VIII sind bereits im Handel erhältlich.

Durch Zugabe von großen Mengen an Saccharose bei der Stabilisierung der angereicherten Konzentrate nach dem bisherigen Stand der Technik bildet sich eine hochviskose und daher schlecht filtrierbare Proteinlösung, die jedoch vor Anwendung als Therapeutikum steril filtriert werden muß. Aus diesem Grund müssen bisher die Konzentrate vor der Sterilfiltration von den Stabilisatorsubstanzen befreit werden. Diese Behandlung ist jedoch mit großem technischen Aufwand und Verlust von Konzentrat verbunden.

Bisher sind Antihämophiliefaktoren wegen der geringen Stabilität in der Lösung nur als lyophilisierte Präparationen erhältlich. Die Lyophilisation ist jedoch ein aufwendiger und energieintensiver Produktionsprozeß.

Außerdem müssen die lyophilisierten Präparate vor Gebrauch in geeigneten Lösungsmitteln aufgenommen werden. Dies ist eine für den Anwender unbegueme Vorbereitung der Therapie.

Aufgabe der vorliegenden Erfindung ist es daher, Faktor VIII in einer leichter verwendbaren, einfacher herstellbaren und stabilen, injizierbaren Form zur Verfügung zu stellen.

Diese Aufgabe wird durch eine stabile injizierbare Lösung gelöst, die einen für die Behandlung am Menschen geeigneten Faktor VIII, natürliche oder synthetische Disaccharide, vorzugsweise Saccharose, in einer Konzentration von 0,1 bis 0,65 mol/l und ein oder mehrere Aminosäuren in einer Konzentration von 0,1 bis 1,0 mol/l enthält.

Als Aminosäure kann Lysin und/oder Glycin verwendet werden.

In einer besonderen Ausführungsform enthält die stabile injizierbare Lösung Ca²⁺-Ionen oder Cl⁻-Ionen. Die Konzentration der Cl⁻-Ionen liegt vorzugsweise bei 0,03 - 0,3 mol/l.

In einer besonders bevorzugten Ausführungsform ist Saccharose in einer Konzentration von 0,4 bis 0,65 mol/l enthalten.

Ein weiterer Gegenstand der Erfindung ist ein Verfahren zur Herstellung einer stabilen injizierbaren Lösung, bei dem ein für die Behandlung am Menschen geeigneter Faktor VIII, ein Disaccharid in einer Konzentration von 0,1 bis 0,65 mol/l, vorzugsweise 0,4 bis 0,65 mol/l Saccharose, und eine oder mehrere Aminosäuren in einer Konzentration von 0,1 bis 1,0 mol/l in Wasser gelöst, steril filtriert und in Ampullen gefüllt werden.

Als Aminosäuren können dabei Lysin und Glycin verwendet werden. Es können zusätzlich Ca²⁺-Ionen oder Cl⁻-Ionen zugesetzt werden.

Diese gebrauchsfertigen Ampullen, in denen Saccharose und eine oder mehrere Aminosäuren zur Stabilsierung von Faktor VIII verwendet werden, können bei 0 - 10 °C vorzugsweise 4 °C über mehrere Wochen stabil gehalten werden. Sie weisen weiterhin den Vorteil auf, daß die an Gerinnungsfaktor VIII angereicherten Plasmafraktionen Saccharose in erheblich geringeren Konzentrationen, als bisher nach dem Stand der Technik üblich, enthalten. Die daraus entstehenden Proteinlösungen sind daher niedrigviskos und leichter filtrierbar, als die nach dem Stand der Technik der EP 0 018 561 stabilisierten hochviskosen Lösungen.

Die folgenden Beispiele zeigen typische Ausführungsformen der Erfindung und des Verfahrens zur Herstellung einer stabilen injizierbaren Lösung von Faktor VIII.

### Vergleichsbeispiel 1:

### Stabilisierung eines Faktor VIII.-Konzentrates

Eine Faktor VIII-haltige Lösung wird nach EP-A 88 108 458.6 bzw. EP-A 88 118 478. 2 gewonnen.

Die gesammelten Faktor VIII-haltigen Fraktionen werden mit 0,9 mol/l Saccharose, 0,25 mol/l Glycin, 0,25 mol/l Lysin und 0,003 mol/l CaCl₂ versetzt und anschließend steril filtiert.

Die Lösung wird bei 4 bis 8°C aufbewahrt. Wie folgende Tabelle zeigt, bleibt die Faktor VIII-Aktivität im Vergleich zu einer nicht-stabilisierten Kontrollösung über Wochen unverändert.

| Wochen | 0 | 1 | 2 | 5 | 10 | 13 |
|---|---|---|---|---|---|---|
| Faktor VIII IU/ml Kontrollösung | 22 | 20 | 11 | - | - | - |
| Faktor VIII IU/ml stabilisierte Lösung | 22 | 22 | 23 | 21 | 23 | 22 |

### Vergleichsbeispiel 2:

### Stabilisierung eines lyophilisierten Faktor VIII-Konzentrates nach Wiederauflösen

Ein Faktor VIII-Konzentrat wird wie unter 1. hergestellt und lyophilisiert. Anschließend wird das Lyophilisat in eimem wässrigen Lösungsmittel mit folgender Zusammensetzung wieder gelöst und steril filtriert:
0,7 mol/l Saccharose
0,5 mol/l Glycin
0,005 mol/l CaCl₂
Die sterile Lösung ist bei 4 bis 8°C haltbar wie unter Beispiel 1 gezeigt.

## Patentansprüche

1. Stabile injizierbare Lösung, dadurch gekennzeichnet, daß sie einen für die Behandlung am Menschen geeigneten Faktor VIII, natürliche oder synthetische Disaccharide in einer Konzentration von 0,1 bis 0,65 mol/l und eine oder mehrere Aminosäuren in einer Konzentration von 0,1 bis 1,0 mol/l enthält.

2. Stabile injizierbare Lösung nach Anspruch 1, dadurch gekennzeichnet, daß sie als Aminosäure Lysin enthält.

3. Stabile injizierbare Lösung nach Anspruch 1 und 2, dadurch gekennzeichnet, daß sie als Aminosäure Glycin enthält.

4. Stabile injizierbare Lösung nach einem der Ansprüche 1 bis 3, dadurch gekennzeichnet, daß zusätzlich Ca²⁺-Ionen oder Cl⁻-Ionen enthalten sind.

5. Stabile injizierbare Lösung nach einem der Ansprüche 1 bis 4, dadurch gekennzeichnet, daß als Disaccharid Saccharose verwendet wird.

6. Stabile injizierbare Lösung nach einem der Ansprüche 1 bis 5, dadurch gekennzeichnet, daß Saccharose in einer Konzentration von 0,4 bis 0,65 mol/l enthalten ist.

7. Verfahren zur Herstellung einer stabilen injizierbaren Lösung, dadurch gekennzeichnet, daß ein für die Behandlung am Menschen geeigneter Faktor VIII, natürliche oder synthetische Disaccharide in einer Konzentration von 0,1 bis 0,65 mol/l und eine oder mehrere Aminosäuren in einer Konzentration von 0,1 bis 1,0 - mol/l in Wasser gelöst, steril filtriert und in Ampullen gefüllt werden.

8. Verfahren nach Anspruch 7, dadurch gekennzeichnet, daß als Aminosäure Lysin verwendet wird.

9. Verfahren nach Anspruch 7 oder 8, dadurch gekennzeichnet, daß als Aminosäure Glycin verwendet wird.

10. Verfahren nach einem der Ansprüche 7 bis 9, dadurch gekennzeichnet, daß zusätzlich Ca²⁺-Ionen zugesetzt werden.

11. Verfahren nach einem der Ansprüche 7 bis 10, dadurch gekennzeichnet, daß als Disaccharid Saccharose verwendet wird.

12. Verfahren nach einem der Ansprüche 7 bis 11, dadurch gekennzeichnet, daß Saccharose in einer Konzentration von 0,4 bis 0,65 mol/l enthalten ist.

## Claims

1. A stable, injectable solution, characterized in that it contains a factor VIII suitable for the treatment of humans, natural or synthetic disaccharides in a concentration of from 0.1 to 0.65 mol/l and one or more amino acids in concentrations of from 0.1 to 1.0 mol/l.

2. The stable, injectable solution according to claim 1, characterized in that it contains lysine as the amino acid.

3. The stable, injectable solution according to claims 1 and 2, characterized in that it contains glycine as the amino acid.

4. The stable, injectable solution according to one of claims 1 to 3, characterized in that it contains, in addition, Ca²⁺ ions or Cl⁻ ions.

5. The stable, injectable solution according to one of claims 1 to 4, characterized in that saccharose is used as the disaccharide.

6. The stable, injectable solution according to one of claims 1 to 5, characterized in that saccharose is contained in a concentration of from 0.4 to 0.65 mol/l.

7. A process for preparing a stable, injectable solution, characterized in that a factor VIII suitable for the treatment of humans, natural or synthetic disaccharides in concentrations of from 0.1 to 0.65 mol/l and one or more amino acids in concentrations of from 0.1 to 1.0 mol/l are dissolved in water, filtered in sterile fashion, and filled into ampoules.

8. The process according to claim 7, characterized in that lysine is used as the amino acid.

9. The process according to claim 7 or 8, characterized in that glycine is used as the amino acid.

10. The process according to one of claims 7 to 9, characterized in that Ca²⁺ ions are added in addition.

11. The process according to one of claims 7 to 10, characterized in that saccharose is used as the disaccharide.

12. The process according to one of claims 7 to 11, characterized in that saccharose is contained in a concentration of from 0.4 to 0.65 mol/l.

## Revendications

1. Solution injectable stable, caractérisée en ce qu'elle contient un facteur VIII approprié à -- un traitement chez l'homme, des disaccharides naturels ou synthétiques à une concentration de 0,1 à 0,65 mole/l et un ou plusieurs amino-acides à une concentration de 0,1 à 1,0 mole/l.

2. Solution injectable stable selon la revendication 1, caractérisée en ce qu'elle contient de la lysine comme amino-acide.

3. Solution injectable stable selon la revendication 1 ou 2, caractérisée en ce qu'elle contient de la glycine comme amino-acide.

4. Solution injectable stable selon l'une des revendications 1 à 3, caractérisée en ce qu'elle contient, en outre, des ions Ca²⁺ ou des ions Cl⁻.

5. Solution injectable stable selon l'une des revendications 1 à 4, caractérisée en ce qu'on utilise du saccharose comme disaccharide.

6. Solution injectable stable selon l'une des revendications 1 à 5, caractérisée en ce qu'elle contient du saccharose à une concentration de 0,4 à 0,65 mole/l.

7. Procédé de préparation d'une solution injectable stable, caractérisé en ce qu'on dissout un facteur VIII approprié à -- un traitement chez l'homme, des disaccharides naturels ou synthétiques à une concentration de 0,1 à 0,65 mole/l et un ou plusieurs amino-acides à une concentration de 0,1 à 1,0 mole/l dans de l'eau, on filtre la solution résultante dans des conditions stériles et on introduit le filtrat dans des ampoules.

8. Procédé selon la revendication 7, caractérisé en ce qu'on utilise la lysine comme amino-acide.

9. Procédé selon la revendication 7 ou 8, caractérisé en ce qu'on utilise la glycine comme amino-acide.

10. Procédé selon l'une des revendications 7 à 9, caractérisé en ce qu'on ajoute, en outre, des ions Ca²⁺.

11. Procédé selon l'une des revendications 7 à 10, caractérisé en ce qu'on utilise du saccharose comme disaccharide.

12. Procédé selon l'une des revendications 7 à 11, caractérisé en ce que du saccharose est contenu à une concentration de 0,4 à 0,65 mole/l.
